# EUROPEAN PATENT APPLICATION

(11) **EP 2 808 813 A1**
(43) Date of publication of application: **03.12.2014**
(21) Application number: 13169675.9
(22) Date of filing: 29.05.2013
(51) Int. Cl.: G06F 19/00

(54) **Medical system and method for authorizing a user to use a medical device of a medical system**

(71) Applicant: Evondos Oy, 24240 Salo (FI)
(72) Inventor: Apell, Mika, 20700 TURKU (FI); Niinistö, Jyrki, 24800 HALIKKO (FI)
(74) Representative: Turun Patenttitoimisto Oy

(57) **Abstract**

The present invention relates to a medical system which comprises a server (101) having a user database (111) that contains information about users of the medical system, and a medical device (102) configured to communicate with the server (101) through a communications network (104). In the medical system the server (101) comprises a test database (112) containing at least one test, the passing of a test being configured to generate an authorization for a user to use the medical device (102), the server (101) is configured to register authorizations on the user database (111), and the medical device (102) is configured to obtain authorizations from the user database (111). The invention also relates to an authorization method.

## Description

### TECHNICAL FIELD OF THE INVENTION

The present invention relates to a medical system and to a method for authorizing a user to use a medical device of a medical system according to the preambles of the appended independent claims.

### BACKGROUND OF THE INVENTION

Various medical devices have been designed for effective prevention, diagnosis, treatment and rehabilitation of illnesses and diseases. These medical devices range from simple thermometers to sophisticated diagnostic imaging equipment and to patient information systems, and can be used by health care workers, patients and/or individuals in a variety of settings including hospitals, clinics and even at home. Some of the medical devices operate as stand-alone units, whereas the others are incorporated in a communications network as parts of medical systems.

The use of a medical device typically requires special knowledge from its user. Such knowledge is conventionally studied from a user manual of the medical device or learned from other users. For some medical devices there may also exist web-based training materials which can be studied using a computer.

A problem associated with known medical devices concerns the difficulty of knowing whether a user has the required knowledge to use a medical device. The use of a medical device can be limited by means of an authentication to a group of users or to only one user, but there is not a way of supervising that the users are competent to use the medical device. The lack of competence may result in errors that can even be fatal to a patient.

### OBJECTIVES OF THE INVENTION

It is the main objective of the present invention to reduce or even eliminate prior art problems presented above.

It is an objective of the present invention to provide a medical system enabling to control the use of a medical device. In more detail, it is an objective of the invention to provide a medical system in which only the competent users are allowed to use a medical device of the medical system. It is also an objective of the invention to provide an easy-to-access learning environment for the users to learn to use a medical device of the medical system correctly and for the system administrators to ensure and maintain sufficient competence level.

It is also an objective of the present invention to provide a method enabling to authorize a competent user to use a medical device.

In order to realise the above-mentioned objectives, the system and the method according to the invention are characterised by what is presented in the characterising parts of the appended independent claims. Advantageous embodiments of the invention are described in the dependent claims.

### DESCRIPTION OF THE INVENTION

A typical medical system according to the invention comprises a server having a user database that contains information about users of the medical system, and a medical device configured to communicate with the server through a communications network. In the typical medical system according to the invention the server comprises a test database containing at least one test, the passing of a test being configured to generate an authorization for a user to use the medical device, the server is configured to register authorizations on the user database, and the medical device is configured to obtain authorizations from the user database.

In the medical system according to the invention the use of the medical device requires an authorization. A user may obtain such an authorization by passing a test. The user's authorization is stored on the user database, from which the medical device receives the authorization.

In the medical system according to the invention the information management is centralized on the server from which the medical device may obtain information relating to the users of the medical device. The medical device communicates with the server over a communications network, such as a LAN, WAN or the Internet. The server may consist of more than one server unit to which the user database and the test database are distributed.

By a medical device is meant any instrument, apparatus, appliance or software that is used alone or in combination, including software specifically for diagnostic or therapeutic purposes that the manufacturer intends for use in human beings. Such devices are used for diagnosis, prevention, monitoring, treatment or alleviation of a disease; for diagnosis, monitoring, treatment, alleviation of or compensation for an injury or a handicap; or for investigation, replacement or modification of the anatomy or of a physiological process. Examples of a medical device are, for example, a blood glucose meter, heart rate monitor, medication dispenser, patient information system and telecare system. A medical device can be a medical hardware and/or software device. A medical device can be a medical software running on a server or a terminal device, such as a personal computer.

The user database contains identification and authorization information of the users of the medical system. All or only some of the users whose information is stored on the user database may have been registered as users of the medical device. The users of the medical device can be patients, caregivers, administrators and/or other health care workers, depending on the type of the medical device. The identification information is used for identifying users who carry out tests and use the medical device. The identification information of a user may contain, for example, an identification code which uniquely identifies the user on the medical system. The identification information of a user may also contain a user name, a user password, additional login key lists containing keys for secured identification, a phone number for sending one time identification keys and/or an electronic ID like an RFID, NFC or magnetic tag or electronic ID card unique IDs. The authorization information of a user contains the user's authorizations like which medical devices of the medical system the user is allowed to use and by which user rights. The user database may also contain other user related information, such as information about the tests a user may carry out and information about the test results and the time of carrying out the tests. The user database can also contain information about the usage of the system or different system parts, usage logs and detected usage errors.

The purpose of a test is to measure a user's competence to use the medical device. The test typically tests matters which are related to certain functions of the medical device. These functions may relate to the hardware and/or the software of the medical device. The tests can be user-specific, so that each of the users of the medical device is associated with a unique test. The tests to be carried out may also be dependent on the user's type, so that for example a caregiver and a health care worker are arranged to carry out different tests. The tests may also have been intended to different user groups, so that each user group is associated with its own test(s). A test typically contains questions, in which case the passing or failing of the test is determined based on the user's answers to the questions. A test may be allowed to be carried out again by a user even though the user already has a valid authorization.

An authorization is arranged to give certain rights to a user. Depending on the test, the authorization may give full or limited rights to use the medical device. In some cases a user may be allowed to carry out a plurality of tests, whereby the user can gain more rights by carrying out the tests one after the other. An authorization may have a time-limited validity, whereby the authorization lapses after a certain time and therefore the test related to the authorization needs to be carried out regularly. A test may also need to be carried out again in cases where the test has been changed or updated. A system administrator may change or update tests to ensure and maintain sufficient competence level or keep the tests updated as the medical system is developed and new types of medical devices are added in the system.

The use of the medical device requires that the user is authenticated. The user may be authenticated, for example, based on user credentials, additional secured identification methods like a key number list or one time key sent to the user's mobile phone or information provided with an electronic key, such as an RFID tag, an NFC tag, or a smartcard. The user can supply the necessary information, for example, using a graphical user interface, or using an electronic key reader of the medical device. Such information is then compared to the identification information obtained from the user database in order to authenticate the user. If the user can be authenticated, it is checked whether the user has an authorization to use the medical device.

The medical system may comprise a plurality of medical devices which are configured to communicate with the server through a communications network. Depending on the application, the number of the medical devices in the medical system can vary from one to thousands of medical devices. The medical devices may be of the same type, in which case the same tests can be used for each medical device. However, if the medical system comprises medical devices of at least two types, the test database typically contains device-specific tests. In other words, in this case each type of the medical device is provided with its own set of tests on the test database. The information about the users who have been registered for each of the medical devices is stored on the user database.

The server may comprise a device database that contains information about the medical devices of the medical system. The device database may contain, for example, the type and identification information of the medical device, required user rights to use the medical device or different features in it and required tests for the users. The device database can also link an individual medical device to a healthcare unit or even individual users.

An advantage of the medical system according to the invention is that it enables to easily control the use of a medical device. In the medical system according to the invention a medical device can only be used by competent users, whereby the usage errors are minimised. The competence of a user is ensured by a test, the passing of which authorizes the user to use the medical device in the medical system. The medical system according to the invention provides an easy-to-access learning environment for the users and a tool for the system administrators to ensure and maintain sufficient competence to use medical devices in the system. The medical system according to the invention enhances patient treatment and safety.

According to an embodiment of the invention the test database contains at least two tests, the passing of which gives different rights to a user. By carrying out different tests, a user may thus obtain different rights to use the medical device. The tests may have to be carried out in a certain order, for example from the easiest test to the most difficult one. Typically the easiest tests are arranged to give the most limited rights to a user, whereas the most difficult tests may even give full rights to the user.

According to an embodiment of the invention the medical system comprises an application configured to enable a user to carry out the at least one test. The application is a software application that is executable on the server and has access to the user database and the test database. The application can also be executable on the medical device and/or a terminal device in connection to the server. The terminal device can be, for example, a personal computer. The application is configured to authenticate a user and to select, based on the authentication, a test to be carried out from the test database. The application is also configured to execute the test, and after the test has been carried out, to determine whether the user has passed or failed the test. The application is configured to register an authorization on the user database, if the test has been passed.

According to an embodiment of the invention the application is executable on the medical device and/or a terminal device. The application comprises a graphical user interface through which a user may carry out a test. The medical device and/or the terminal device are provided with a display screen. The terminal device, which can be, for example, a laptop or tablet computer, or a mobile phone, is configured to communicate with the server over a communications network.

According to an embodiment of the invention the application is configured to authenticate a user based on user credentials or information provided with an electronic key. The necessary information can be obtained, for example, using the graphical user interface, or using an electronic key reader of the medical device or the terminal device. An electronic key can be, for example, an RFID tag, an NFC tag, or a smartcard.

According to an embodiment of the invention the medical device is configured to store the authorizations obtained from the user database in its local memory. This enables to check, without accessing the server, whether a user has an authorization to use the medical device. The authorization information on the memory of the medical device is updated automatically when the authorization information of the users of the medical device stored on the user database is changed. Because the use of the medical device requires authentication of a user prior to determining whether the user has an authorization, the identification information of the users of the medical device is also stored in the memory of the medical device.

According to an embodiment of the invention the test comprises training material and questions. The passing or failing of the test is determined based on the user's answers to the questions. The training material may contain videos and electronic documents, which can be studied while answering the questions. The questions and the training material are typically related to a medical device in the medical system.

According to an embodiment of the invention the medical device is a medication dispenser. By a medication dispenser is meant a device which can dispense proper dosages of medications at prescribed times. The medications are prepackaged into medication packages, which are provided with labels that may contain information about the patient, the content of the package, and the taking time of the dosage. Typically, the medication packages are connected together to form a strip, from which medication packages are dispensed one by one.

The present invention also relates to a method for authorizing a user to use a medical device of a medical system that comprises a server having a user database containing information about users of the medical system. The method according to the invention comprises authenticating a user, selecting, based on the authentication, a test from a test database located on the server, the passing of the test authorizing the user to use the medical device, and carrying out the test. The method according to the invention further comprises determining whether the user has passed or failed the test, and in a case where the user has passed the test, sending an authorization from the server to the medical device to authorize the user to use the medical device.

A user is authenticated by comparing the information supplied by the user with the identification information of the user database. The authentication may be performed at the server, or at the medical device or a terminal device with which the user carries out the test. Based on the authentication, the test to be carried out is selected from the test database. The test typically contains questions to which the user must provide answers. The answers can be stored on the user database. Based on the answers it is determined whether the user has passed or failed the test. Preferably, the authorization is stored on the user database, from which the authorization is sent to the medical device over a communications network.

According to an embodiment of the invention the user is authenticated based on user credentials or information provided with an electronic key.

According to an embodiment of the invention the method comprises in a case where a test has been updated or removed, deleting the authorizations related to the original test. If a test has been updated, a user must pass the updated test or the updated parts of the test in order to use the medical device. A test may be updated or removed by a system administrator who has access to the test database.

According to an embodiment of the invention the method comprises in a case where a predetermined number of usage errors of the medical device has been detected, deleting user's authorization to use the medical device. The number of usage errors that are allowed before the user's authorization is deleted, depend on the type of the medical device as well as the role of the user. Typically, the more the user has rights, the lower is the number of the usage errors allowed. In some cases, no usage errors are allowed.

Other cases where users lose their authorizations are, for example, when the authorizations have a time-limited validity. The authorizations may be valid for only a certain time after which the corresponding tests must be passed again.

The exemplary embodiments of the invention presented in this text are not interpreted to pose limitations to the applicability of the appended claims. The verb "to comprise" is used in this text as an open limitation that does not exclude the existence of also unrecited features. The features recited in the dependent claims are mutually freely combinable unless otherwise explicitly stated.

The exemplary embodiments presented in this text and their advantages relate by applicable parts to the system as well as the method according to the invention, even though this is not always separately mentioned.

Being computer-related, it can be appreciated that the components disclosed herein may be implemented in hardware, software, or a combination of hardware and software. Software components may be in the form of computer-readable program code stored in a computer-readable storage medium such as memory, mass storage device, or removable storage device. For example, a computer-readable medium may comprise computer-readable code for performing the function of a particular component. Likewise, computer memory may be configured to include one or more components, which may then be executed by a processor. Components may be implemented separately in multiple modules or together in a single module.

### BRIEF DESCRIPTION OF THE DRAWINGS

The features which are considered as characteristic of the invention are set forth in particular in the appended claims. The invention itself, however, both as to its construction and its method of operation, together with additional objects and advantages thereof, will be best understood from the description of specific embodiments when read in connection with the accompanying drawings.
- Fig. 1: illustrates a medical system according to an embodiment of the invention, and
- fig. 2: illustrates a flow diagram of an authorization method according to an embodiment of the invention.

### DETAILED DESCRIPTION OF THE DRAWINGS

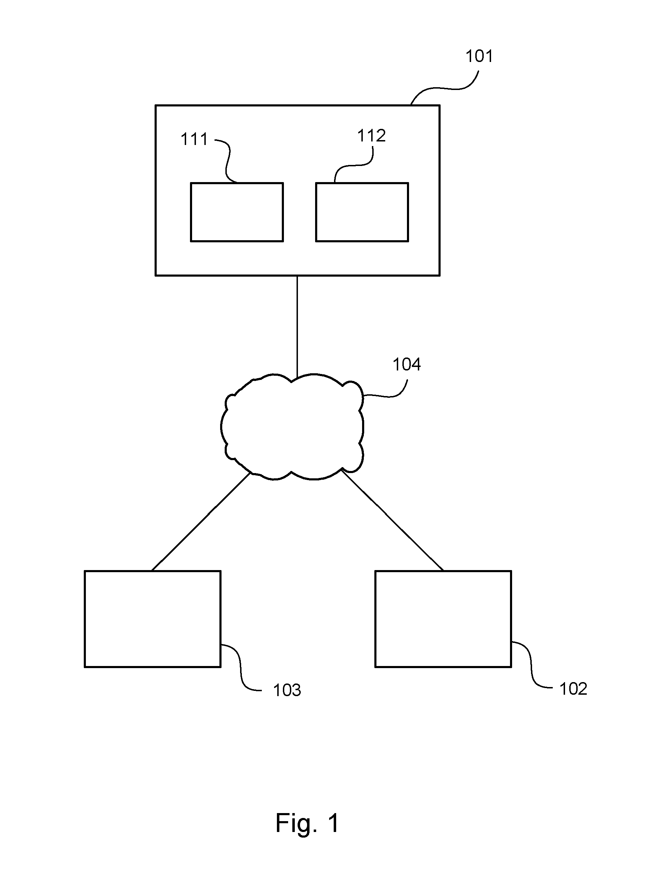

Fig. 1 illustrates a medical system according to an embodiment of the invention. The medical system comprises a server 101 that has a user database 111 and a test database 112. The user database 111 contains information about the user or users and the test database 112 contains one or more tests that the user or users must pass in order to be authorized to use a medical device 102. A test may be carried out using the medical device 102 or a terminal device 103. The server 101, the medical device 102 and the terminal device 103 each have a connection to a communications network 104 that enables the communication between the user database 111, the test database 112, the medical device 102 and the terminal device 103. Indeed, with the medical device 102 or the terminal device 103 the user accesses a test in the test database 112 through the communications network 104 and takes the test. If the user passes the test, an authorization is stored on the user database 111. Then when the user wishes to use the medical device 102, the medical device 102 either already has the authorization or requests it from the user database 111.

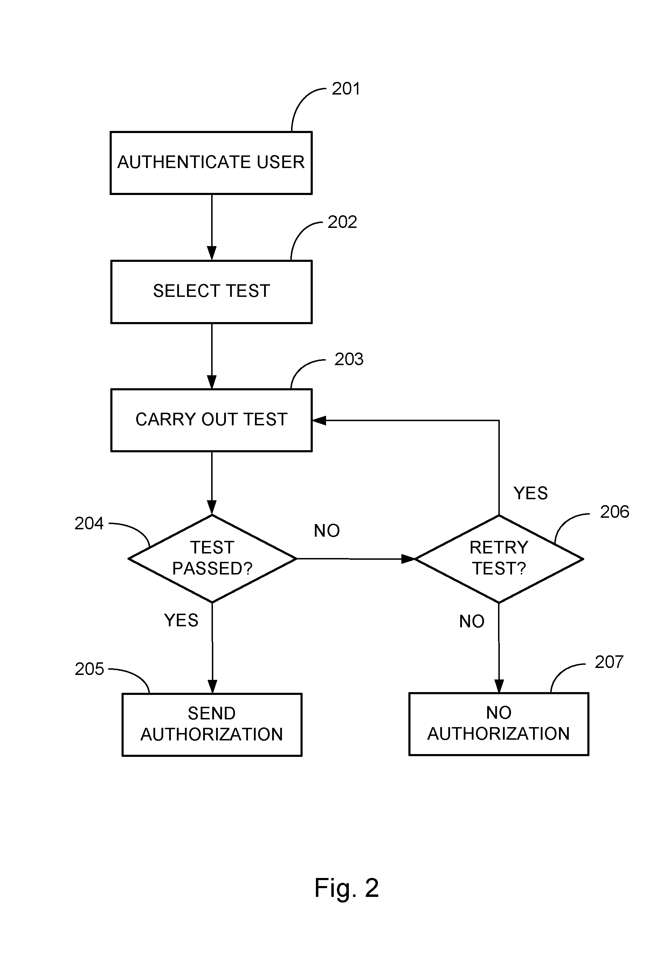

Fig. 2 illustrates a flow diagram of an authorization method according to an embodiment of the invention. In the method, the user is first authenticated (step 201). Thereafter, based on this authentication, a test is selected from the test database (step 202) and presented to the user. The user then carries out the test (step 203), after which it is determined whether the user has passed or failed the test (step 204). If the user has passed said test, an authorization is sent to a medical device to authorize the user to use the medical device (step 205). If the user has failed the test, the user may decide to carry out the test again (step 206). If the user decides to try again, the method is continued at step 203. If the user decides not to try again, the user does not obtain an authorization (step 207).

Only advantageous exemplary embodiments of the invention are described in the figures. It is clear to a person skilled in the art that the invention is not restricted only to the examples presented above, but the invention may vary within the limits of the claims presented hereafter. Some possible embodiments of the invention are described in the dependent claims, and they are not to be considered to restrict the scope of protection of the invention as such.

## Claims

1. A medical system, comprising:
- a server having a user database that contains information about users of the medical system, and
- a medical device configured to communicate with the server through a
communications network;
**characterised in that**:
- the server comprises a test database containing at least one test, the passing of a test being configured to generate an authorization for a user to use the medical device,
- the server is configured to register authorizations on the user database, and
- the medical device is configured to obtain authorizations from the user database.

2. The medical system according claim 1, **characterised in that** the test database contains at least two tests, the passing of which gives different rights to a user.

3. The medical system according to claim 1 or 2, **characterised in that** the medical system comprises an application configured to enable a user to carry out the at least one test.

4. The medical system according to claim 3, **characterised in that** the application is executable on the medical device and/or a terminal device.

5. The medical system according to claim 3 or 4, **characterised in that** the application is configured to authenticate a user based on user credentials or information provided with an electronic key.

6. The medical system according to any of the preceding claims, **characterised in that** the medical device is configured to store the authorizations obtained from the user database in its local memory.

7. The medical system according to any of the preceding claims, **characterised in that** the test comprises training material and questions.

8. The medical system according to any of the preceding claims, **characterised in that** the medical device is a medication dispenser.

9. A method for authorizing a user to use a medical device of a medical system that comprises a server having a user database containing information about users of the medical system, **characterised in that** the method comprises:
- authenticating a user,
- selecting, based on the authentication, a test from a test database located on the server, the passing of the test authorizing the user to use the medical device,
- carrying out the test,
- determining whether the user has passed or failed the test, and
- in a case where the user has passed the test, sending an authorization from the server to the medical device to authorize the user to use the medical device.

10. The method according to claim 9, **characterised in that** the user is authenticated based on user credentials or information provided with an electronic key.

11. The method according to claim 9 or 10, **characterised in that** the method comprises:
- in a case where a test has been updated or removed, deleting the authorizations related to the original test.

12. The method according to any of claims 9 to 11, **characterised in that** the method comprises:
- in a case where a predetermined number of usage errors of the medical device has been detected, deleting user's authorization to use the medical device.
